# FASCICULE DE BREVET EUROPEEN

(11) **EP 1 517 861 B1**
(45) Date de publication et mention de la délivrance du brevet: **15.02.2006**
(21) Numéro de dépôt: 03748190.0
(22) Date de dépôt: 27.06.2003
(51) Int. Cl.: C02F 1/48, A61L 2/00

(54) **PROCEDE DE TRAITEMENT THERMIQUE PAR INDUCTION D'UNE CANALISATION D'EAU SANITAIRE ET SYSTEME POUR SA MISE EN OEUVRE**
VERFAHREN ZUR INDUZIERTEN WÄRMEBEHANDLUNG VON EINER WASSERVERSORGUNGSROHRLEITUNG UND EINRICHTUNG ZUR DURCHFÜRUNG DES VERFAHRENS
METHOD FOR INDUCTION THERMAL TREATMENT OF A DOMESTIC WATER SUPPLY PIPE AND SYSTEM THEREFOR

(30) Priorité: 28.06.2002 FR 0208060
(43) Date de publication de la demande: 30.03.2005
(73) Titulaire: SA Deschamps-Lathus, F-86361 Chasseneuil Poitou (FR)
(72) Inventeur: LATHUS, Laurent, F-86180 Avanton (FR); PRIOTON, Claude, F-86440 Migne Auxances (FR); DELPIERRE, Fabrice, F-77115 Blandy les Tours (FR); GUITTON, Rodolphe, F-77166 Grisy Suisnes (FR); BAILLI, Didier, F-37220 Crouzille (FR)
(74) Mandataire: Abello, Michel
(86) Numéro de dépôt international: PCT/FR2003/002008
(87) Numéro de publication internationale: WO 2004/002899

(56) Documents cités:
- EP-A- 0 553 377
- EP-A- 0 685 987
- US-A- 5 241 147
- US-A- 6 056 884
- PATENT ABSTRACTS OF JAPAN vol. 1999, no. 08, 30 juin 1999 (1999-06-30) & JP 11 076083 A (MIWA SHIRO), 23 mars 1999 (1999-03-23)

## Description

La présente invention est relative à un procédé de traitement thermique des canalisations contenant de l'eau stagnante d'un réseau de distribution d'eau sanitaire afin d'altérer des agents contaminants, et à un système permettant de mettre en oeuvre ce procédé de traitement.

Il est connu que les systèmes humides sont des lieux de développement d'agents contaminants. En particulier, les légionelles forment une classe particulière de bactéries nocives pour la santé humaine. L'une des principales sources d'infection est constituée par les circuits de distribution d'eau chaude sanitaire. Les infections sont plus fréquentes et plus graves en cas de diminution des défenses de l'utilisateur. C'est pourquoi le secteur hospitalier est particulièrement exposé et sensible à ce problème. Depuis la fin des années 70 et l'identification de la légionelle, des efforts incessants ont été réalisés pour mettre en place des procédures et des dispositifs dans le but de décontaminer les installations d'eau sanitaire et de prévenir le développement de cette bactérie.

La prolifération des légionelles est favorisée par une eau à une température comprise entre 20°C et 45°C. La prolifération est favorisée par une eau stagnante. Elle est également favorisée par la présence d'autres micro-organismes, comme des protozoaires, des algues, des amibes, d'autres bactéries etc. qui forment ensemble un agglomérat dénommé biofilm qui se dépose en pellicule, dont la texture est grasse, sur les parois des canalisations en particulier. La prolifération des légionelles est favorisée par la présence de certains sels minéraux. Ce sont ces facteurs organiques et minéraux favorisant la prolifération des légionelles que nous nommons agents contaminants au travers de cette demande.

Les autorités responsables de la santé publique recommandent à la fois des actions de prévention et des actions de décontamination des installations de distribution d'eau sanitaire.

Parmi les recommandations de prévention, la planification des installations est préconisée afin de limiter les parties de l'installation favorables au développement d'agents contaminants. Il s'agit de favoriser les réseaux de petite dimension où l'on supprime au maximum les canalisations propices à la stagnation de l'eau. L'installation une fois réalisée, la meilleure méthode pour prévenir la prolifération est de garantir, pour le circuit de distribution d'eau chaude, une température d'au moins 60 °C au réservoir et de 50°C aux robinets. En outre l'eau froide doit rester au-dessous de 20°C. C'est la raison pour laquelle on recommande une bonne isolation entre le circuit d'eau chaude et le circuit d'eau froide. Tout ceci ne dispense pas de veiller également au bon fonctionnement et à l'hygiène de l'installation, en particulier après une période d'arrêt de l'installation, par un rinçage complet de l'installation en faisant s'écouler l'eau par les robinets, et par un nettoyage avec de l'eau de Javel.

Parmi les modes de décontamination, outre les méthodes peu efficaces comme l'utilisation de lumière ultraviolette, l'ozonisation ou l'ionisation, on utilise habituellement les méthodes de choc thermique et de choc chloré.

La procédure conseillée en France pour la désinfection du réseau d'eau sanitaire par choc thermique consiste à augmenter la température jusqu'à obtenir une eau chaude à 70°C à la sortie de tous les robinets, puis à laisser couler l'eau pendant 30 mn.

La méthode de choc chloré conseillée en France pour la désinfection du réseau d'eau sanitaire consiste à ajouter du chlore sous forme d'eau de Javel jusqu'à une concentration de 15 mg/l à maintenir pendant 24 heures puis à vidanger et à rincer l'installation pour éliminer le chlore résiduel.

On combine parfois ces deux méthodes en réalisant un choc chloré suivi d'un choc thermique.

Bien que la longueur des canalisations contenant de l'eau stagnante soit réduite en optimisant l'architecture du réseau, ces canalisations existeront toujours dans une installation de distribution d'eau sanitaire. La séparation thermique des réseaux d'eau chaude et d'eau froide n'est également pas facile à mettre en oeuvre car les canalisations des deux réseaux empruntent les mêmes gaines de service.

Le principal désavantage des méthodes thermiques ou chimiques de prévention ou de décontamination, c'est qu'il s'agit de traiter l'ensemble du réseau.

Les valeurs thermiques idéales (<20°C et >45°C) qu'il faut maintenir à travers l'ensemble du réseau et en permanence, représente une contrainte forte et une consommation importante d'énergie.

De plus, les méthodes de décontamination nécessitent la mise hors service de l'installation durant toute la durée du procédé.

La décontamination par choc thermique est la méthode de choix dans les réseaux d'eau potable, mais la faisabilité reste douteuse puisqu'il faut ouvrir l'ensemble des robinets de l'installation et s'assurer que l'eau est à 70°C. Cette technique présente l'inconvénient majeur d'utiliser beaucoup d'eau et d'énergie. Elle provoque également l'usure de certains matériaux non thermorésistants.

La décontamination par choc chloré provoque la corrosion des conduites (risque d'oxydation important sur toutes les tuyauteries métalliques), la libération de produits dits cancérigènes. Le dosage exact du chlore présente également un risque. Et si l'on fait suivre ce choc chloré d'un choc thermique il faut s'assurer de bien séparer ces deux opérations car les hautes températures favorisent la formation de substances toxiques.

Enfin la décontamination ne suffit pas. Il faut que des procédures périodiques préventives soient mises en place pour éviter la recolonisation de l'installation. Ainsi, par exemple, la concentration requise en chlore afin d'éviter la recolonisation est de 2 mg/l, ce qui est difficilement compatible avec un standard d'eau potable à 0.1 mg/l.

Finalement pour des motifs économiques et parce que les hôpitaux sont souvent la cible d'épidémies importantes, les autres bâtiments sont rarement mentionnés dans les documents officiels. Or les canalisations de distribution d'eau sanitaire d'un hôtel, d'une maison de retraite ou d'une habitation particulière peuvent être contaminées par les légionelles, et tout particulièrement juste en amont de tout point d'utilisation.

L'invention a pour but d'éliminer les inconvénients précités et de proposer un procédé de traitement thermique local, c'est à dire qui permet de ne traiter que les parties de l'installation où la bactérie est susceptible de se développer, à savoir les canalisations contenant de l'eau stagnante.

L'invention a également pour but de proposer un procédé de traitement thermique dont le coût en eau et en énergie est réduit.

Encore un autre but est que le procédé de traitement thermique soit efficace, sûr et simple à mettre en oeuvre.

L'invention a aussi pour but de proposer un dispositif fiable permettant de réaliser ce procédé de traitement thermique.

Un but de l'invention est également de proposer un dispositif qui soit intégré à l'installation lors de sa planification ou bien ajouté à celle-ci ultérieurement, et qui permette éventuellement une gestion technique centralisée à distance par enregistrement, contrôle etc. des variables physiques comme la température par exemple.

L'invention a encore pour but de proposer un dispositif qui permet au procédé d'être utilisable comme moyen de décontamination, mais surtout comme moyen de prévention qui peut être appliqué périodiquement.

Le dispositif proposé de traitement a également pour but de réduire les coûts de revient d'installation et d'utilisation par rapport aux autres méthodes de traitement. Et ceci pour qu'éventuellement ce dispositif soit utilisé non seulement dans les hôpitaux mais également dans les hôtels, les maisons de retraite, les habitations particulières etc.

A cet effet, l'invention à pour objet un procédé de traitement thermique d'une canalisation d'eau sanitaire contenant de l'eau stagnante dans le but d'éliminer des agents contaminants, qui peuvent être des sels minéraux tels que le calcaire, un biofilm contenant des micro-organismes, tels que des amibes ou des bactéries, des légionelles en particulier, caractérisé en ce que ladite canalisation comporte sur toute sa longueur au moins une couche conductrice continue en matériau conducteur ohmique du courant électrique, et que le procédé consiste à :
a) relier deux portions de ladite couche conductrice par un élément de liaison en matériau conducteur du courant électrique afin de former une boucle fermée de conducteur
b) engendrer un flux magnétique variable à travers ladite boucle fermée, pour induire une énergie électrique dans ladite boucle fermée, tout ou partie de ladite énergie électrique se dissipant par effet Joule en chaleur dans ladite couche conductrice entre lesdites deux portions, de façon que tout ou partie de ladite chaleur se transmette et chauffe ladite eau stagnante initialement à une température dite de consigne.
c) maintenir le flux magnétique variable tant que la température de l'eau stagnante n'a pas atteint une valeur de seuil prédéterminée dite température de traitement
d) adapter le flux magnétique variable pour maintenir la température de l'eau stagnante à une valeur supérieure ou égale à ladite température de traitement pendant une durée prédéterminée, dite durée de traitement, suffisante pour altérer les agents contaminants
e) interrompre le flux magnétique variable après ladite durée de traitement.

Avantageusement, ce procédé peut comprendre, avant l'étape a), une étape d'isolement de la canalisation contenant de l'eau stagnante du reste du circuit de distribution d'eau sanitaire, en fermant une vanne en aval et/ou une vanne en amont de la canalisation à traiter, et de préférence de manière automatique, afin de limiter ou d'interdire pendant le traitement la distribution de l'eau contenue dans ladite canalisation, et après l'étape e) une étape de rétablissement de la distribution d'eau sanitaire au travers de ladite canalisation en ouvrant de nouveau la ou lesdites vannes, une fois que la température de l'eau stagnante est revenue à la température de consigne.

De même, le procédé peut comprendre également après l'étape e), une étape de purge de la canalisation contenant de l'eau stagnante consistant à faire circuler de l'eau sanitaire dans ladite canalisation, afin d'éliminer par écoulement les agents contaminants altérés. L'invention a également pour objet un système de mise en oeuvre du procédé précédent. Ce système comporte une canalisation d'eau sanitaire comportant sur toute sa longueur au moins une couche conductrice continue en matériau conducteur ohmique du courant électrique, un élément de liaison en matériau conducteur du courant électrique reliant deux portions de ladite couche conductrice afin de former une boucle fermée de conducteur, et un dispositif pour engendrer un flux magnétique variable à travers ladite boucle fermée.

Ladite canalisation conductrice comporte de préférence au moins un tuyau à âme métallique intercalée entre une couche interne et une couche externe d'un isolant électrique, les deux portions reliées par ledit élément de liaison correspondant à des portions du tuyau qui ont été dénudées d'une couche externe d'isolant pour faire apparaître l'âme métallique et permettre un contact électrique entre l'âme métallique et l'élément de liaison.

Ladite canalisation conductrice comprend avantageusement plusieurs tuyaux à âme métallique reliés par des raccords, la continuité électrique de l'ensemble de la canalisation étant assurée au niveau desdits raccords par des éléments de liaison électriques intermédiaires reliant chacune des extrémités dénudées, adjacentes à un raccord, des tuyaux situés de part et d'autre dudit raccord.

Dans un autre mode de réalisation ladite canalisation conductrice comprend plusieurs tuyaux à âme métallique reliés par des raccords conducteurs comportant une couche en un matériau conducteur du courant électrique.

De préférence, au moins un raccord conducteur comporte au moins un épaulement permettant un contact électrique entre d'une part la couche conductrice du raccord conducteur et d'autre part une partie de l'âme métallique, située à l'extrémité du tuyau de manière adjacente au raccord, dénudée d'au moins une de ses couches isolantes.

Dans un autre mode de réalisation, au moins un raccord conducteur comporte un élément annulaire conducteur externe muni de pointes radialement saillantes vers l'intérieur, qui traversent la couche externe d'isolant du tuyau et viennent en contact avec l'âme métallique du tuyau, assurant la continuité électrique entre ladite âme métallique dudit tuyau et ladite couche conductrice dudit raccord conducteur.

Avantageusement, la canalisation comporte plusieurs tuyaux présentant une seconde âme métallique formant l'élément de liaison placée entre ladite couche extérieure et la première âme métallique et séparée de celle-ci par une couche supplémentaire d'un isolant du courant électrique.

Ledit dispositif pour engendrer un flux magnétique variable à travers ladite boucle est de préférence un transformateur à coeur magnétique dont le bobinage primaire est alimenté par un courant variable et dont le bobinage secondaire est constitué par ladite boucle fermée de conducteur.

Avantageusement, l'un au moins des raccords conducteurs, dit raccord transformateur, comporte, d'une part, une paroi interne où se situe ladite couche conductrice du courant, et d'autre part, une paroi externe définissant avec la paroi interne un espace annulaire entre les deux parois, dans lequel est logé le coeur magnétique ainsi que le bobinage primaire dudit transformateur.

Dans un autre mode de réalisation ledit raccord transformateur comporte dans sa paroi externe une seconde couche conductrice du courant électrique en liaison électrique avec ladite seconde âme métallique du tuyau et telle que les deux couches conductrices du raccord transformateur soient isolées électriquement l'une de l'autre.

L'invention sera mieux comprise, et d'autres buts, détails, caractéristiques et avantages de celle-ci apparaîtront plus clairement au cours de la description suivante de plusieurs modes de réalisation particuliers de l'invention, donnés uniquement à titre illustratif et non limitatif, en référence aux dessins annexés.

Sur ces dessins :
- la figure 1 est une représentation schématique du système selon l'invention.
- la figure 2 est une coupe axiale partielle de deux tuyaux à âme métallique, d'un raccord conducteur, et d'un raccord d'extrémité conducteur, faisant partie du système selon une première variante de l'invention.
- la figure 2A est une vue agrandie d'un détail de la figure 2, comme indiqué par le cercle IIA, représentant la liaison réalisée au moyen d'une bague à picots entre le tuyau à âme métallique et le raccord conducteur.
- la figure 3 est une coupe axiale partielle de deux tuyaux à double âme métallique, de deux raccords d'extrémité conducteurs, et d'un raccord transformateur, selon une seconde variante de l'invention.

Les éléments de la seconde variante identiques ou analogues à ceux de la première variante portent les mêmes chiffres de référence augmentés d'une centaine.

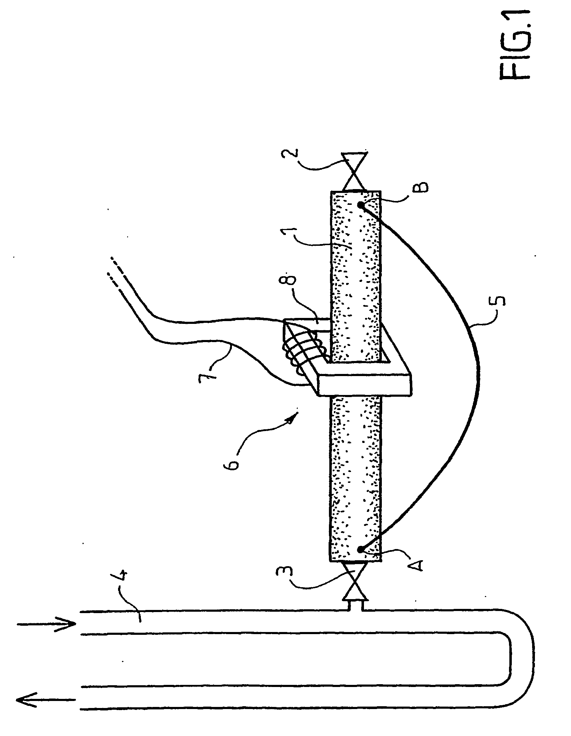

La figure 1 représente, de manière schématique, le système de traitement thermique par induction selon l'invention. Une canalisation 1, contenant de l'eau stagnante, est située entre une vanne aval 2, un robinet par exemple, et une vanne amont 3. L'eau de la canalisation 1 est admise au travers de la vanne amont 3 en provenance d'une canalisation 4 de distribution d'eau sanitaire. Dans le cas particulier d'un circuit de distribution d'eau chaude sanitaire, circule en permanence dans la canalisation 4 une eau en provenance d'un générateur (non représenté), tel qu'une chaudière, et maintenue à une température d'environ 60°C, appelée température de consigne.

La canalisation 1 doit être traitée thermiquement entre deux points A et B situés à chacune des deux extrémités de la canalisation 1 afin d'altérer les agents contaminants qui ont pu se développer entre ces points sur la surface intérieure de la canalisation 1.

La canalisation 1 comporte sur toute sa longueur une couche continue conductrice, métallique par exemple ou plus généralement une couche continue en un matériau conducteur ohmique du courant électrique.

Un fil électrique 5, jouant le rôle d'un élément de liaison en matériau conducteur du courant électrique, est en contact électrique avec la couche conductrice de la canalisation 1 en chacun des points A et B. En A ou en B, la connexion entre le fil électrique 5 et la couche conductrice de la canalisation 1 est réalisée, lorsque cela est nécessaire, en dénudant localement la canalisation 1, d'une couche d'isolant par exemple, aux points A et B afin de faire apparaître la couche conductrice. Les points A et B sont ainsi mis au même potentiel électrique. De A à B le long de la couche conductrice de la canalisation 1 et de B à A le long du fil électrique 5, une boucle fermée de conducteur, notée ABA, a ainsi été formée.

Sur la figure 1, un coeur magnétique 8, de section rectangulaire creuse entoure la canalisation 1 en un point situé entre les points A et B. Le fil électrique 5 passant à l'exrtérieur du coeur magnétique 8. De manière équivalente, le coeur magnétique 8 pourrait être situé en n'importe quel autre point de la boucle fermée de conducteur ABA, à condition qu'une de ses branches soit située à l'extérieur de la boucle fermée ABA et qu'une autre de ses branches soit située à l'intérieur de la boucle fermée ABA. Un bobinage primaire 7, faisant plusieurs spires, par exemple 222 spires, est enroulé autour du coeur magnétique 8. Ce bobinage primaire 7 est alimenté par un générateur de courant (non représenté sur la figure 1) qui délivre un courant variable, par exemple un courant sinusoïdal de fréquence 50 Hz ou 60 Hz et d'amplitude 3000 A.

Finalement, le bobinage primaire 7, le coeur magnétique 8 et la boucle fermée de conducteur ABA forment un transformateur de courant 6. Le bobinage secondaire du transformateur 6 n'est autre que la boucle fermée de conducteur ABA, faisant une seule spire autour du coeur magnétique 8. Dans un autre mode de réalisation, la boucle fermée de conducteur ABA pourrait faire plusieurs spires autour du coeur magnétique 8.

Lorsque le bobinage primaire 7 est parcouru par un courant variable, un champ magnétique variable est produit le long de l'axe des N1 spires. Ce champ magnétique variable est concentré et guidé à l'intérieur du coeur magnétique 8.

Or, selon les lois de l'induction, lorsqu'un champ magnétique variable passe à travers une surface s'appuyant sur une boucle de conducteur, un courant est induit dans cette boucle. C'est ce principe qui est mis en jeu lorsque le champ magnétique variable est guidé de manière à traverser la boucle fermée de conducteur ABA formant une spire autour du coeur magnétique 8. Le flux du champ magnétique variable à travers une surface s'appuyant sur la boucle fermée de conducteur ABA induit un courant électrique dans la boucle fermée de conducteur ABA.

De manière annexe, la couche continue conductrice de la canalisation 1 étant un conducteur possédant une cavité, le courant électrique induit prend la forme d'un courant induit à la surface extérieure de la couche continue conductrice par effet Joule, appelé encore courant de Foucault.

Une partie de l'énergie électrique induite, transportée par le courant induit, se dissipe par effet Joule dans la couche de conducteur ohmique de la canalisation 1. Une partie de la chaleur ainsi produite chauffe la couche continue conductrice, se transmet à toute la paroi de la canalisation 1 et finalement permet de chauffer l'eau stagnante contenue dans la canalisation 1.

La température de l'eau s'élève d'une température initiale, à une température dite de traitement.

Dans un premier temps, le courant variable est telle qu'il permet un échauffement du système composé de la canalisation 1 entre les points A et B et de l'eau stagnante contenue.

Dans un second temps, lorsque la température de traitement est atteinte, le courant variable d'alimentation du bobinage primaire 7 est adapter de manière à maintenir l'eau stagnante à cette température de traitement, pendant une durée, dite durée de traitement. Cette durée et cette température de traitement sont définies en fonction de la canalisation à traiter, des matériaux qui la composent et de son diamètre entre autres choses, afin d'altérer au maximum les agents contaminants et de réaliser un traitement efficace.

A titre d'exemple, des tests ont montré qu'une durée de 1 heure et une température de 90°C détruisait 100% des légionelles.

Finalement, après cette phase, le courant variable d'alimentation du bobinage primaire 7 du transformateur 8 est coupé. La température du système composé de la canalisation 1 entre les points A et B et de l'eau stagnante contenue redescend à une température proche de la température initiale. Ceci peut se faire par simple diffusion de la chaleur vers le milieu extérieur à la canalisation

Pour des raisons de sécurité, durant ce traitement thermique, la canalisation 1 est isolée du reste du circuit de distribution d'eau sanitaire. En particulier la vanne aval 2 est commandée en position fermée, bloquée afin d'interdire la distribution d'une eau dont la température n'est pas connue, et peut être très élevée.

Afin de permettre au système constitué de la canalisation 1 et de l'eau stagnante contenue de redescendre plus vite à une température proche de la température initiale, et de limiter le temps d'indisponibilité de la distribution d'eau sanitaire, et afin d'éliminer de la canalisation les agents contaminants altérés en suspension dans l'eau stagnante de la canalisation 1, une phase de purge sera avantageusement réalisée en faisant circuler de l'eau au travers la canalisation 1. A cet effet, les deux vannes 2 et 3 sont commandées en position ouverte pendant la phase de purge.

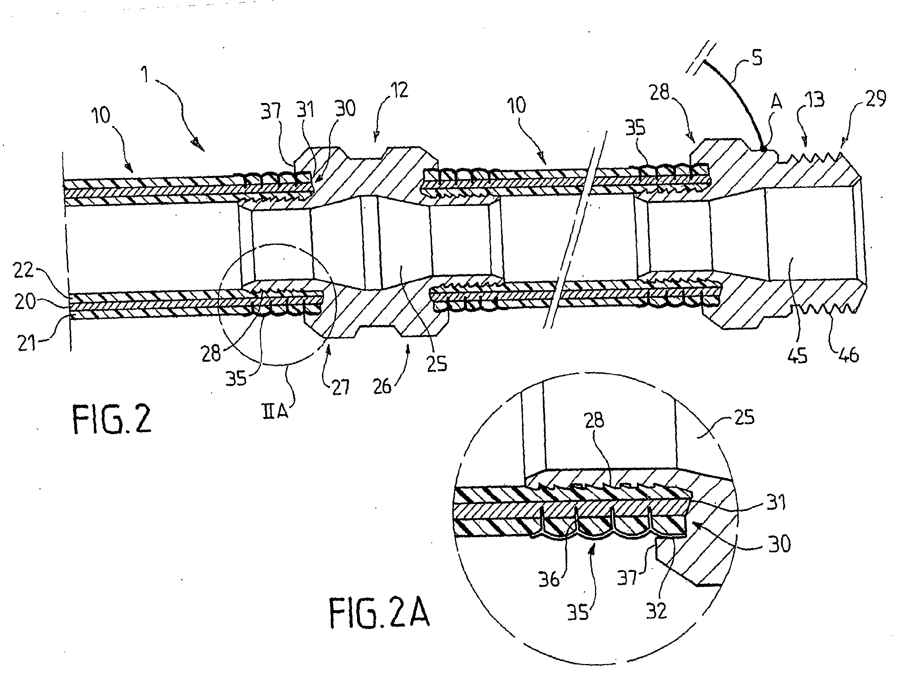

La figure 2 représente une coupe axiale partielle d'un mode de réalisation particulier du système selon une première variante de l'invention.

La canalisation 1 peut comporter plusieurs tuyaux 10 reliés entre eux par des raccords conducteurs intermédiaires 12 et reliés à des vannes par exemple aux extrémités de la canalisation 1 par des raccords conducteurs d' extrémité 13.

Chaque tuyau 10 est constitué d'une âme métallique 20 centrale, en aluminium par exemple et plus généralement en un matériau conducteur ohmique du courant électrique, située entre une couche intérieure 22 et une couche extérieure 21 en un matériau isolant du courant électrique, en Polyéthylène par exemple. L'âme métallique 20 centrale constitue une partie de la couche continue de conducteur de la canalisation 1 et permet de dissiper par effet Joule le courant électrique induit. La chaleur produite dans l'âme métallique 20 se transmet au travers de la couche intérieure isolante 22 à l'eau contenue dans la canalisation 1.

Le raccord intermédiaire conducteur 12, en métal par exemple, comporte un axe principal de symétrie de révolution. Il présente un trou central 25 de même axe, dont le diamètre peut varier le long de cet axe principal, pour la communication de l'eau entre les deux tuyaux 10 situés de part et d'autre du raccord intermédiaire conducteur 12. Le raccord intermédiaire conducteur 12 comporte deux portions 26 et 27 identiques, symétriques l'une de l'autre par rapport à un plan perpendiculaire à l'axe principal. Chacune des portions 26 et 27 comporte une jupe 28 globalement cylindrique dont le diamètre externe est légèrement supérieur au diamètre interne du tuyau 10, et munie sur sa surface externe de crans en V, afin de réaliser un engagement hermétique par accrochage des crans avec la couche interne d'isolant 22 du tuyau 10, lorsque la jupe 28 du raccord intermédiaire conducteur 12 est emmanchée à l'intérieur de l'extrémité du tuyau 10 à raccorder. En position raccordée, la portion d'extrémité du tuyau 10 vient en butée sur le fond 31 d'une gorge annulaire 30 ménagée en vis à vis sur une face radiale 37 de la portion 27 du raccord intermédiaire conducteur 12. La section de cette gorge annulaire 30 est globalement rectangulaire et sa largeur est identique ou légèrement inférieure à l'épaisseur du tuyau 10.

En se référant maintenant à la figure 2A, est représentée plus en détail la jonction entre le tuyau 10 à âme métallique 20 et la partie 27 du raccord intermédiaire conducteur 12. La continuité électrique, le long de la canalisation 1, entre l'âme métallique 20 et le raccord intermédiaire conducteur 12 est assurée au moyen d'une bague de sertissage 35. Celle-ci, réalisée en matériau conducteur, est, à une extrémité, emmanchée en contact électrique avec le raccord intermédiaire conducteur 12 par contact avec le bord extérieur 32 de la gorge annulaire 30. La bague de sertissage 35 comporte, à l'autre extrémité, des parties saillantes radialement vers l'intérieur qui, dans ce mode de réalisation particulier, prennent la forme de picots 36. Au moment du sertissage de la bague de sertissage 35, les picots 36 percent la couche extérieure isolante 21 du tuyau 10, et viennent en contact électrique avec l'âme métallique 20 du tuyau 10.

De la même manière, sur la figure 2, le raccord d'extrémité conducteur 13, en métal par exemple, comporte un axe principal de symétrie de révolution. Il présente un trou central 45 de même axe, dont le diamètre peut varier le long de cet axe principal, pour la communication de l'eau entre le tuyau 10 et un élément de l'installation de distribution d'eau sanitaire, une vanne par exemple. Le raccord d'extrémité conducteur 13 comporte deux portions 28 et 29. La portion 28 et la portion 27 du raccord intermédiaire conducteur 12 sont identiques à la fois dans leur structure, dans leur mode de raccordement au tuyau 10 pour assurer la continuité hydraulique et la continuité électrique. La partie 29 peut comporter une surface extérieure filetée 46 pour son raccordement à une vanne par exemple.

Aux extrémités A ou B de la canalisation 1, lorsque le raccord d'extrémité est métallique, ce dernier peut être directement relié au fil électrique 5, ce qui permet d'éviter de dénuder localement, de la couche externe d'isolant 21, l'extrémité du tuyau 10 proche de l'extrémité A ou B de la canalisation 1.

Lorsque les raccords intermédiaires sont en matériau conducteur ohmique du courant électrique, il est également possible de traiter thermiquement les raccords et notamment d'altérer le biofilm qui s'est déposé à l'interface sur la paroi interne du trou 25 du raccord intermédiaire conducteur 12.

Si ces raccords intermédiaires ne sont pas métalliques, il est alors nécessaire de relier les tuyaux 10 adjacents de part et d'autre du raccord intermédiaire, par exemple par un fil métallique externe jouant le rôle d'une liaison externe intermédiaire, pour assurer la continuité électrique le long de la canalisation 1. Mais dans ce cas le raccord n'est pas directement traité. Le volume d'eau qu'il contient peut être traité indirectement par diffusion de la chaleur des volumes d'eau, contenus dans les tuyaux 10 adjacents au raccord intermédiaire, portés à la température de traitement.

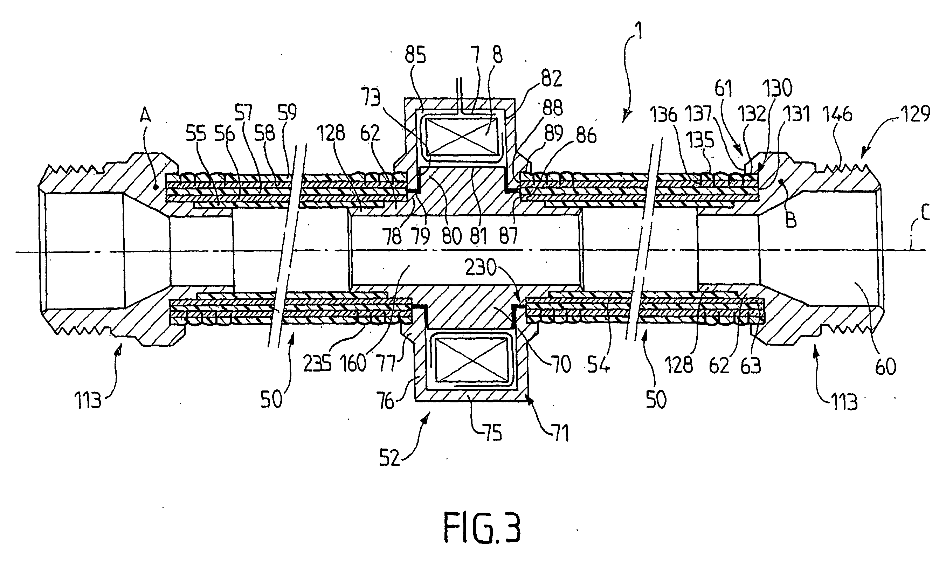

La figure 3 est une coupe axiale partielle d'une canalisation 1 comportant deux tuyaux 50 à double âme métallique, de deux raccords conducteurs d'extrémité 113, et d'un raccord transformateur 52 central, selon une seconde variante de l'invention.

Le tuyau 50 comporte, en se déplaçant radialement de l'axe principal vers l'extérieur, une couche interne d'isolant 55, une première âme métallique 56, une couche intermédiaire d'isolant 57, une seconde âme métallique 58 et une couche extérieure d'isolant 59.

La première âme métallique 56, en aluminium par exemple, joue le rôle de la couche continue de conducteur de la canalisation 1 qui, par effet Joule, va dissiper le courant induit en une chaleur permettant de chauffer l'eau sanitaire contenue dans le tuyau 50.

La seconde âme métallique 58, en aluminium par exemple, joue le rôle de l'élément de liaison entre les extrémités de la canalisation 1 entre lesquelles le traitement est appliqué.

Les différentes couches isolantes 55, 57 et 59, en polyéthylène par exemple, isolent électriquement les âmes métalliques les unes des autres et du milieu extérieur et intérieur.

Le raccord conducteur d'extrémité 113, en métal par exemple, de la figure 3, est similaires aux raccords d'extrémité décrits précédemment sur la figure 2. Il comporte un axe principal de symétrie de révolution. Il présente un trou central 60 de même axe, dont le diamètre peut varier le long de cet axe principal, pour la communication de l'eau entre le tuyau 50 et un élément de l'installation de distribution d'eau sanitaire, une vanne par exemple. Le raccord conducteur d'extrémité 113 comporte deux portions 61 et 129. La portion 129 peut comporter une surface extérieure filetée 146 pour son raccordement à une vanne par exemple. La portion 61 comporte une jupe 128 globalement cylindrique dont le diamètre externe est légèrement supérieur au diamètre interne du tuyau 50, et munie sur sa surface externe de crans en V, afin de réaliser un engagement hermétique par accrochage des crans avec la couche interne d'isolant 55 du tuyau 50, lorsque la jupe 128 de la portion 61 du raccord conducteur d'extrémité 113 est emmanchée à l'intérieur de l'extrémité du tuyau 50 à raccorder.

A la base de 1a jupe 128, la portion 61 du raccord conducteur d'extrémité 113 comporte un épaulement 62, dont l'épaisseur est légèrement supérieure à l'épaisseur de la couche interne d'isolant 55. Une portion annulaire située à l'extrémité de la surface intérieure 54 du tuyau 50 est dénudée de la couche interne d'isolant 55. Ainsi, en position raccordée, la première âme métallique 56, sur une portion égale à la portion annulaire dénudée, vient en contact électrique avec le raccord conducteur d'extrémité 113 en s'appuyant sur la surface cylindrique externe 63 de l'épaulement 62.

En position raccordée, une portion d'extrémité du tuyau 50 vient en butée sur le fond 131 d'une gorge annulaire ménagée en vis à vis sur une face radiale 137 de la portion 61 du raccord conducteur d'extrémité 113. La section de cette gorge annulaire 130 est globalement rectangulaire et sa largeur est identique à l'épaisseur cumulée des quatre couches externes 56, 57, 58, et 59 du tuyau 50.

La continuité électrique, entre la seconde âme métallique 58 et le raccord conducteur d'extrémité 113 est assurée au moyen d'une bague de sertissage 135. Celle-ci, réalisée en matériau conducteur, est à une extrémité emmanchée en contact électrique avec le raccord conducteur d'extrémité 113 par contact avec le bord extérieur 132 de la gorge annulaire 130. La bague de sertissage 135 comporte, à l'autre extrémité, des parties saillantes radialement vers l'intérieur qui, dans ce mode de réalisation particulier, prennent la forme de picots 136. Au moment du sertissage de la bague de sertissage 135, les picots 136 percent la couche extérieure isolante 59 du tuyau 50, et viennent en contact électrique avec la seconde âme métallique 58 du tuyau 50, sans toucher la couche 56.

Au travers de la portion 61 du raccord conducteur d'extrémité 113, les deux âmes métalliques 56 et 58 du tuyau 50 sont placées en continuité électrique. Aux extrémités A et B de la canalisation 1, les points de contact électrique, entre la couche continue de conducteur de la canalisation 1 qui dans cette variante de l'invention est constituée par la première âme métallique 56 et l'élément de liaison qui dans cette variante de l'invention est constitué par la seconde âme métallique 59, ont été ainsi crées.

Le raccord transformateur 52 comporte deux pièces 70 et 71, à symétrie de révolution selon le même axe, en matériau conducteur. La pièce 71 entoure la pièce 70 et est séparée de celle-ci par une garniture d'isolant électrique 73.

La pièce 71 est annulaire et présente une section en C ouverte vers son axe, avec une paroi axialement extérieure 75. Les parois latérales 76 de la pièce 71 comporte des portions saillantes 77 axialement vers l'extérieur situées à une certaine distance du bord intérieur de la paroi latérale 76. A titre d'exemple, la pièce 71 est constituée de deux demi-coquilles pour pouvoir être montée sur la pièce 70.

La pièce 70, symétrique par rapport à un plan médian orthogonal à l'axe principal, présente d'un trou central 160 de même axe que la pièce 70, dont le diamètre peut varier le long de cet axe principal, pour la communication de l'eau entre les tuyaux 50 placés de part et d'autre du raccord transformateur 52.

La pièce 70 comporte une jupe 128 et un premier épaulement 62 largement décrits plus haut dans le cas particulier du raccord conducteur d'extrémité 113.

A la base de ce premier épaulement 62, la pièce 70 comporte un second épaulement 78 dont l'épaisseur est inférieure à l'épaisseur cumulée de la couche intermédiaire d'isolant 57 et de la première âme métallique 56. La surface interne du bord intérieur de la paroi latérale 76 de la pièce 71 vient appuyer la garniture d'isolant électrique 73 contre la surface cylindrique externe 79 de ce second épaulement 78.

A la base de ce second épaulement 78, la pièce 70 comporte un troisième épaulement 80 formant corps central de la pièce 70. L'épaisseur de ce troisième épaulement 80 est inférieure à la profondeur de l'évidement intérieur de la pièce 71, de telle sorte que, lorsque les pièces 70 et 71 sont positionnées l'une avec l'autre, la surface axialement externe 81 de ce troisième épaulement 80 et la surface interne 82 de la pièce 71 forment un espace annulaire 85.

La surface axiale externe 86 du premier épaulement 62 de la pièce 70, la surface radiale externe 87 du second épaulement 78 de la pièce 70, la surface radiale externe 88 du bord intérieur de la paroi latérale 76 de la pièce 71 ainsi que la surface axiale interne 89 de la saillie 77 de la paroi latérale 76 de la pièce 71, forment, lorsque les pièces 70 et 71 sont positionnées l'une avec l'autre, les parois d'une gorge annulaire 230.

En position raccordée, la portion d'extrémité du tuyau 50 vient en butée sur le fond de la gorge annulaire 230. La distance entre les parois axiales de cette gorge annulaire 230 est identique ou inférieure à l'épaisseur cumulée des quatre couches externes 56, 57, 58, et 59 du tuyau 50.

La continuité électrique, entre la seconde âme métallique 58 et la pièce 71 du raccord transformateur 52 est assurée au moyen d'une bague de sertissage 235. Celle-ci, réalisée en matériau conducteur, est à une extrémité emmanchée en contact électrique avec la pièce 71 par contact avec le bord extérieur de la gorge annulaire 230 qui est en fait la surface axiale interne 89 de la saillie 77 de la paroi latérale 76 de la pièce 71. A l'autre extrémité, la bague de sertissage 235 comporte des picots qui assurent le contact avec la seconde âme métallique 58 du tuyau 50 comme cela a déjà été mentionné.

Ainsi la continuité de la couche interne de la canalisation 1 est réalisée en reliant les premières âmes métalliques 56 des tuyaux 50 situés de part et d'autre du raccord transformateur 52 au moyen de la pièce 70 intérieure conductrice du raccord transformateur 52. La continuité de l'élément de liaison est réalisée en reliant les secondes âmes métalliques 58 des tuyaux 50 situés de part et d'autre du raccord transformateur 52 au moyen de la pièce 71 extérieure conductrice du raccord transformateur 52.

Dans l'espace annulaire 85, est logé le coeur magnétique 8 torique du transformateur. Le coeur magnétique 8 est entouré sur tout son périmètre du bobinage primaire 7 du transformateur. Le bobinage primaire 7, isolé des pièces 70 et 71 conductrices, est alimenté par un courant variable à partir d'un générateur (non représenté).

Le courant variable induit un champ magnétique qui est dirigé selon une direction perpendiculaire à l'axe C des tuyaux. Ce champ magnétique, guidé par le coeur magnétique 8 torique engendre un courant induit dans la pièce 70 conductrice intérieure du raccord transformateur 52, les premières âmes métalliques 56 des tuyaux 50 et les raccords d'extrémité 51. Ces éléments constitutifs de la couche continue de conducteur de la canalisation1 étant mis au même potentiel aux points A et B par les secondes âmes métalliques 58 des tuyaux 50 et la pièce 71 extérieure conductrice du raccord transformateur 52, le courant induit se dissipe par effet Joule. La chaleur produite permet de porter l'eau sanitaire contenue dans les tuyaux 50 et le raccord transformateur 52 à la température de traitement.

Cette température maintenue pendant une période de traitement permet d'altérer les légionnelles et de désagréger tout ou partie du biofilm, qui est une pellicule de texture grasse déposée sur les surfaces internes des tuyaux 50 et des raccords 51, 52.

Dans les modes de réalisation de l'invention présentés en détail ci-dessus, il n'a été fait mention que de canalisation rectiligne. Cet aspect de l'invention mérite d'être précisé.

Le principe de l'induction sur lequel se fonde la présente invention nécessite seulement la présence d'un circuit conducteur dont les deux extrémités sont au même potentiel, pour qu'un courant électrique soit induit dans ce circuit. Par la suite les lois générales de l'électricité sont appliquées au courant induit dans ce circuit ; s'il y a un élément conducteur ohmique, la loi de Joule est appliquée ; si le circuit se divise en deux branches parallèles, le courant circulant dans chaque branche peut être connu en fonction des paramètres de chacune des branches, la loi de division des courants étant appliquée ; etc.

Le circuit fermé le plus simple est bien évidemment la boucle fermée de conducteur mentionnée plus haut. Mais l'invention s'applique par exemple à une canalisation possédant un premier tuyau à âme métallique raccordé à deux autres tuyaux au moyen d'un raccord intermédiaire conducteur en forme de T, à condition que les extrémités des trois tuyaux soient connectées par un élément de liaison les plaçant au même potentiel. Mais les courants circulant dans les différents tuyaux n'étant pas les mêmes, si ceux-ci possèdent les mêmes caractéristiques physiques, la température du volume l'eau stagnante dans chacun d'eux sera différente.

Des études scientifiques ayant pour sujet le traitement thermique par induction selon l'invention ont été menées.

Ces études ont d'abord permis de mieux comprendre le mécanisme conduisant à l'efficacité de l'invention. Il s'avère que le traitement thermique par induction permet, dans un premier temps, à la surface intérieure du tuyau d'atteindre une température élevée de l'ordre de 100°C. Cette élévation brusque de la température, accompagnée d'une dilation de la surface et d'effets magnétiques secondaires dépendants de la fréquence du courant induit, permet de décrocher le tartre et le biofilm à l'intérieur duquel se protège les bactéries. Puis, dans un second temps, par convection thermique, l'ensemble du liquide contenu dans 1a canalisation à traiter est porté à une température élevée conduisant à la destruction de la faune contenue dans le liquide.

Ces études ont ensuite permis de mettre en évidence que pour un traitement curatif, porter le contenu de la canalisation à une température de 85 à 90°C pendant une durée de 30 mn, comme le préconise la circulaire ministérielle française DGS/SD7A/SD5C-DHOS/E4 n°2002/243 relative à la prévention du risque lié aux légionelles dans les établissements de santé, ne suffit pas. De préférence, un traitement à une température de 80°C pendant 60 mn semble mieux convenir pour détruire les légionelles. De préférence encore, un traitement à une température de 90°C pendant 60 mn permet de détruire la totalité des bactéries et des amibes mésophiles.

Par ailleurs, dans le cas d'un traitement préventif, le protocole visant à prévenir la contamination des tuyaux et consistant à traiter à une température de 90°C pendant 10 mn une fois par semaine les tuyauteries d'un réseau, a été validé par ces études.

Il a été mis en évidence que le traitement thermique par induction ne détériore pas la structure du tuyau. Par exemple, pour un tuyau en cuivre, aucun ion métallique n'a été détecté dans le liquide contenu dans la canalisation traitée. En conséquence, la composition de l'eau distribuée n'est pas modifiée par le traitement thermique par induction.

En conséquence, et pour le cas particulier du monde hospitalier dans lequel les normes sont les plus drastiques, la demanderesse propose une série de valeurs numériques. L'énergie électrique utilisée au primaire est avantageusement celle du secteur avec une fréquence de 50 ou de 60 Hz et une tension de 220V. L'intensité dans le circuit secondaire constitué par la boucle continue de conducteur est de 3000 A, mais sous une tension très faible variant entre 1 et 10 V. Cette faible différence de potentiel assure la parfaite sécurité des utilisateurs.

Pour limiter les émissions de rayonnements électromagnétiques par la boucle elle-même, dans le but de se conformer aux normes NF EN 60601-1-1 et 1-1-2 relatives aux appareils médicaux et C18-600 relative aux champs électromagnétiques basses fréquences, des inducteurs spéciaux ayant un rendement élevé de l'ordre de 98% sont de préférence utilisés. De plus, les éléments électriques de liaison nécessaires à la formation de la boucle continue de conducteur doivent suivre au plus prêt le chemin défini par le tuyau à traiter. Le tuyau comportant une double âme métallique est alors particulièrement bien adapté.

Pour donner un ordre d'idée, le traitement d'un tuyau ayant un diamètre de 16 mm, une épaisseur de 4 mm et une longueur de 16 m consomme une puissance électrique inférieure à 0,55 kWh durant un traitement curatif.

La demanderesse a réalisé des simulations de l'installation d'un dispositif de traitement thermique sur l'ensemble d'un réseau de distribution d'eau chaude sanitaire, soit pour le cas d'un réseau préexistant, soit pour le cas d'un réseau équipé dès sa conception. Ces simulations conduisent à penser que les coûts d'installation sont faibles et amortis sur les premières années d'exploitation du réseau, étant donnés les faibles coûts de maintenance du dispositif de traitement selon l'invention, sa faible consommation énergétique ainsi que sa consommation nulle en eau et en produit chimique.

Enfin, le traitement thermique par induction d'un réseau de distribution d'eau chaude sanitaire peut être entièrement contrôlé et automatisé. La capacité de traçabilité du traitement thermique par induction est en parfaite adéquation avec la circulaire mentionnée ci-dessus.

Il est à noter que le traitement thermique par induction, s'il a été décrit en détail pour le cas des canalisations des installations de distribution d'eau chaude sanitaire dans le milieu hospitalier, peut également être mis en oeuvre dans tous les bâtiments sensibles (maison de retraite, laboratoires, etc.), dans des lieux publics de distribution d'eau (piscines, fontaines publiques etc.) ou encore dans des dispositifs de ventilation et/ou de climatisation dans lesquels de l'eau risque de stagner et/ou d'être projetée dans l'atmosphère sous forme d'aérosol.

Incidemment, un onduleur placé dans le circuit électrique et apte à modifier la fréquence du courant induit permet d'utiliser le dispositif de traitement thermique par induction en tant que moyen pour lutter contre le dépôt de calcaire dans les canalisations.

Bien que l'invention ait été décrite en liaison avec plusieurs modes de réalisation particuliers, il est bien évident qu'elle n'y est nullement limitée et qu'elle comprend tous les équivalents techniques des moyens décrits ainsi que leurs combinaisons si celles-ci entrent dans le cadre de l'invention.

## Revendications

1. Procédé de traitement thermique d'une canalisation d'eau sanitaire contenant de l'eau stagnante dans le but d'éliminer des agents contaminants, qui peuvent être des sels minéraux tels que le calcaire, un biofilm comportant des micro-organismes, tels que des amibes ou des bactéries, des légionelles en particulier, **caractérisé en ce que** ladite canalisation (1) comporte sur toute sa longueur au moins une couche conductrice continue (20 ; 56) en matériau conducteur ohmique du courant électrique, et que le procédé consiste à :
a) relier deux portions de ladite couche conductrice par un élément de liaison en matériau conducteur du courant électrique afin de former une boucle fermée de conducteur
b) engendrer un flux magnétique variable à travers ladite boucle fermée, pour induire une énergie électrique dans ladite boucle fermée, tout ou partie de ladite énergie électrique se dissipant par effet Joule en chaleur dans ladite couche conductrice entre lesdites deux portions, de façon que tout ou partie de ladite chaleur se transmette et chauffe ladite eau stagnante initialement à une température dite de consigne.
c) maintenir le flux magnétique variable tant que la température de l'eau stagnante n'a pas atteint une valeur de seuil prédéterminée dite température de traitement
d) adapter le flux magnétique variable pour maintenir la température de l'eau stagnante à une valeur supérieure ou égale à ladite température de traitement pendant une durée prédétenninée, dite durée de traitement, suffisante pour altérer les agents contaminants
e) interrompre le flux magnétique variable après ladite durée de traitement.

2. Procédé selon la revendication 1, **caractérisé en ce qu'**il comprend, avant l'étape a), une étape d'isolement de la canalisation (1) contenant de l'eau stagnante du reste du circuit de distribution d'eau sanitaire (4), en fermant une vanne en aval (2) et/ou une vanne en amont (3) de la canalisation à traiter, afin de limiter ou d'interdire pendant le traitement la distribution de l'eau contenue dans ladite canalisation, et après l'étape e) une étape de rétablissement de la distribution d'eau sanitaire au travers de ladite canalisation en ouvrant de nouveau la ou lesdites vannes (2, 3), une fois que la température de l'eau stagnante est revenue à la température de consigne.

3. Procédé selon l'une des revendications 1 et 2, **caractérisé en ce qu'**il comprend après l'étape e), une étape de purge de la canalisation (1) contenant de l'eau stagnante consistant à faire circuler de l'eau sanitaire dans ladite canalisation, afin d'éliminer par écoulement les agents contaminants altérés.

4. Système de mise en oeuvre du procédé selon l'une des revendications 1 à 3, **caractérisé en ce qu'**il comporte une canalisation (1) d'eau sanitaire comportant sur toute sa longueur au moins une couche conductrice continue (20 ; 56) en matériau conducteur ohmique du courant électrique, un élément de liaison (5 ; 59, 71) en matériau conducteur du courant électrique reliant deux portions (A, B) de ladite couche conductrice afin de former une boucle fermée (ABA) de conducteur, et un dispositif pour engendrer un flux magnétique variable à travers ladite boucle fermée.

5. Système selon la revendication 4, **caractérisé en ce que** ladite canalisation conductrice comporte au moins un tuyau (10 ; 50) à âme métallique (20 ; 56) intercalée entre une couche interne et une couche externe d'un isolant électrique (21, 22 ; 55, 59), les deux portions reliées par ledit élément de liaison correspondant à des portions du tuyau qui ont été dénudées d'une couche d'isolant pour faire apparaître l'âme métallique et permettre un contact électrique entre l'âme métallique et l'élément de liaison.

6. Système selon la revendication 5, **caractérisé en ce que** ladite canalisation conductrice comprend plusieurs tuyaux (10 ; 50) à âme métallique reliés par des raccords, la continuité électrique de l'ensemble de la canalisation étant assurée au niveau desdits raccords par des éléments de liaison électriques intermédiaires reliant chacune des extrémités dénudées, adjacentes à un raccord, des tuyaux situés de part et d'autre dudit raccord.

7. Système selon la revendication 5, **caractérisé en ce que** ladite canalisation conductrice comprend plusieurs tuyaux (10 ; 50) à âme métallique reliés par des raccords conducteurs (12, 28 ; 52 , 113) comportant une couche en un matériau conducteur du courant électrique.

8. Système selon la revendication 7, **caractérisé en ce qu'**au moins un raccord conducteur (70 ; 113) comporte au moins un épaulement (62) permettant un contact électrique entre d'une part la couche conductrice du raccord conducteur et d'autre part une partie de l'âme métallique (56), située à l'extrémité du tuyau de manière adjacente au raccord, dénudée d'au moins une de ses couches isolantes (55).

9. Système selon la revendication 7, **caractérisé en ce qu'**au moins un raccord conducteur (12, 13) comporte un élément annulaire conducteur externe (35) muni de pointes radialement saillantes vers l'intérieur (36), qui traversent la couche externe d'isolant (21) du tuyau (10) et viennent en contact avec l'âme métallique (20) du tuyau, assurant la continuité électrique entre ladite âme métallique dudit tuyau et ladite couche conductrice dudit raccord conducteur.

10. Système selon l'une des revendications 5 à 9, **caractérisé en ce que** la canalisation comporte plusieurs tuyaux (50) présentent une seconde âme métallique (58) formant l'élément de liaison placée entre ladite couche extérieure (59) et la première âme métallique (56) et séparée de celle-ci par une couche supplémentaire (57) d'un isolant du courant électrique.

11. Système selon l'une des revendications 4 à 10, **caractérisé en ce que** ledit dispositif pour engendrer un flux magnétique variable à travers ladite boucle (ABA) est un transformateur (6) à coeur magnétique (8) dont le bobinage primaire (7) est alimenté par un courant variable et dont le bobinage secondaire est constitué par ladite boucle fermée de conducteur.

12. Système selon les revendications 7 et 11 en combinaison, **caractérisé en ce que** l'un au moins des raccords conducteurs (52), dit raccord transformateur, comporte d'une part une paroi interne (70) où se situe ladite couche conductrice du courant, et d'autre part une paroi externe (71) définissant avec la paroi interne un espace annulaire (85) entre les deux parois, dans lequel est logé le coeur magnétique (8) ainsi que le bobinage primaire (7) dudit transformateur (6).

13. Système selon les revendications 10 et 12 en combinaison, **caractérisé en ce que** ledit raccord transformateur (52) comporte dans sa paroi externe (71) une seconde couche conductrice du courant électrique en liaison électrique avec ladite seconde âme métallique (58) du tuyau (50) et telle que les deux couches conductrices du raccord transformateur soient isolées électriquement l'une de l'autre.

## Claims

1. Method for thermal treatment of a domestic water supply pipe containing stagnant water in order to eliminate contaminating agents, which may be mineral salts such as limestone, a biofilm comprising microorganisms, such as amoebae or bacteria, legionellae in particular, **characterized in that** said pipe (1) comprises over its entire length at least one continuous conducting layer (20; 56) made of ohmic material conducting electric current, and that the method consists in:
a) connecting two portions of said conducting layer via a linking element made of material conducting electric current in order to form a closed conducting loop
b) generating a variable magnetic flux through said closed loop, in order to induce an electric energy in said closed loop, some or all of said electric energy being dissipated by Joule heating in said conducting layer between said two portions, such that some or all of said heat is transmitted and heats said stagnant water initially to a temperature known as the setpoint
c) maintaining the variable magnetic flux so long as the temperature of the stagnant water has not reached a predetermined threshold value known as the treatment temperature
d) adapting the variable magnetic flux in order to maintain the temperature of the stagnant water at a value equal to or greater than said treatment temperature for a predetermined period, known as the treatment period, sufficient to impair the contaminating agents
e) interrupting the variable magnetic flux after said treatment period.

2. Method according to Claim 1, **characterized in that** it comprises, before step a), a step of isolating the pipe (1) containing the stagnant water from the rest of the domestic water distribution circuit (4), by closing a valve downstream (2) and/or a valve upstream (3) of the pipe to be treated, in order to limit or prevent during the treatment the distribution of the water contained in said pipe, and, after step e), a step of reestablishing the distribution of domestic water through said pipe by reopening said valve or valves (2, 3), once the temperature of the stagnant water is returned to the setpoint temperature.

3. Method according to either of Claims 1 and 2, **characterized in that** it comprises, after step e), a step of purging the pipe (1) containing the stagnant water consisting in causing domestic supply water to flow in said pipe, in order to clear away the impaired contaminating agents by discharge.

4. System of implementing the method according to one of Claims 1 to 3, **characterized in that** it comprises a domestic water supply pipe (1) comprising over its entire length at least one continuous conducting layer (20; 56) made of ohmic material conducting electric current, a linking element (5; 59, 71) made of material conducting electric current conrecting two portions (A, B) of said conducting layer in order to form a closed conducting loop (ABA), and a device to generate a variable magnetic flux through said closed loop.

5. System according to Claim 4, **characterized in that** said conducting pipe comprises at least one tube (10; 50) with a metal core (20; 56) inserted between an inner layer and an outer layer of an electric insulator (21, 22; 55, 59), the two portions connected by said linking element corresponding to portions of the tube that have been stripped of an insulating layer to expose the metal core and allow an electric contact between the metal core and the linking element.

6. System according to Claim 5, **characterized in that** said conducting pipe comprises several tubes (10; 50) with metal core connected by connectors, the electric continuity of the whole pipe being provided at said connectors by intermediate electric linking elements connecting each of the stripped ends, adjacent to a connector, of the tubes situated either side of said connector.

7. System according to Claim 5, **characterized in that** said conducting pipe comprises several tubes (10; 50) with metal core connected by conducting connectors (12, 28; 52, 113) comprising a layer made of a material conducting electric current.

8. System according to Claim 7, **characterized in that** at least one conducting connector (70; 113) compriees at least one shoulder (62) allowing an electric contact between on the one hand the conducting layer of the conducting connector and on the other hand a portion of the metal core (56), situated at the end of the tube adjacent to the connector, stripped of at least one of its insulating layers (55).

9. System according to Claim 7, **characterized in that** at least one conducting connector (12, 13) comprises an outer conducting annular element (35) furnished with spikes projecting radially inwards (36) which pass through the outer layer of insulation (21') of the tube (10) and make contact with the metal core (20) of the tube, providing electric continuity between said metal core of said tube and said conducting layer of said conducting connector.

10. System according to one of Claims 5 to 9, **characterized in that** the pipe comprises several tubes (50) having a second metal core (58) forming the linking element placed between said outer layer (59) and the first metal core (56) and separated from the latter by an additional layer (57) of an electric current insulator.

11. System according to one of Claims 4 to 10, **characterized in that** said device for generating a variable magnetic flux through said loop (ABA) is a transformer (6) with magnetic core (8) whose primary winding (7) is supplied with a variable current and whose secondary winding consists of said closed conducting loop.

12. System according to Claims 7 and 11 in combination, **characterized in that** at least one of the conducting connectors (52), called the transformer connectoc, comprises on the one hand an inner wall (70) where said current conducting layer is situated, and on the other hand an outer wall (71) defining with the inner wall an annular space (85) between the two walls, in which is housed the magnetic core (3) and the primary winding (7) of said transformer (6).

13. System according to Claims 10 and 12 in combination, **characterized in that** said transformer connector (52) comprises in its outer wall (71) a second layer conducting the electric current in electrical connection with said second metal core (58) of the tube (50) and such that the two conducting layers of the transformer connector are electrically insulated one from the other.

## Patentansprüche

1. Verfahren zur thermischen Behandlung einer Sanitärwasserleitung, welche stagnierendes Wasser enthält, um Verunreinigungen zu eliminieren, bei denen es sich um Mineralsalze, wie beispielsweise Kalk, einen biologischen Film, der Mikroorganismen, wie beispielsweise Amöben oder Bakterien, insbesondere Legionellen, enthält, **dadurch gekennzeichnet, dass** die Leitung (1) auf ihrer gesamten Länge wenigstens eine kontinuierliche leitfähige Schicht (20; 56) aus einem den elektrischen Strom ohmisch leitenden Material aufweist, und dass das Verfahren die folgenden Schritte umfasst:
a) Verbinden von zwei Abschnitten der leitfähigen Schicht durch ein Verbindungselement aus einem den elektrischen Strom leitenden Material zur Bildung einer geschlossenen Leiterschleife,
b) Erzeugen eines variablen magnetischen Flusses durch die geschlossenen Schleife, um in der geschlossenen Schleife eine elektrische Energie zu induzieren, wobei die elektrische Energie in der leitfähigen Schicht zwischen den beiden Abschnitten durch den Jouleschen Effekt vollständig oder teilweise so in Wärme dissipiert, dass die Wärme vollständig oder teilweise auf das anfänglich auf eine Soltwerttemperatur befindliche stagnierende Wasser übertragen wird und dieses erwärmt,
c) Aufrechthalten des variablen magnetischen Flusses, solange die Temperatur des stagnierenden Wassers nicht einen als Behandlungstemperatur bezeichneten Grenzwert erreicht hat,
d) Anpassung des variablen magnetischen Flusses, um die Temperatur des stagnierenden Wassers während eines als Behandlungsdauer bezeichneten Zeitraums, der ausreicht, um die Verunreinigungen zu verändem, auf einem Wert zu halten, der größer oder gleich der Behandlungstemperatur ist,
e) Unterbrechen des variablen magnetischen Flusses nach Ablauf der Behandlungsdauer.

2. Verfahren gemäß Anspruch 1, **dadurch gekennzeichnet, dass** es vor dem Schritt a) einen Schritt der Isolierung der das stagnierende Wasser enthaltenden Leitung (1) von dem restlichen Sanitärwasserkreislauf (4) umfasst, wobei man ein stromabwärts der zu behandelnden Leitung liegendes Ventil (2) und/oder ein stromaufwärts der zu behandelnden Leitung liegendes Ventil (3) schließt, um während der Behandlung einen Weitertransport des in der Leitung enthaltenen Wassers einzuschränken oder zu verhindern, und dass das Verfahren nach dem Schritt e) einen Schritt der Wiederherstellung der Weitertransports des Sanitärwassers durch die Leitung umfasst, wobei man das Ventil oder die Ventile (2, 3) wieder öffnet, sobald die Temperatur des stagnierenden Wassers auf die Sollwerttemperatur zurückgegehrt ist.

3. Verfahren gemäß einem der Ansprüche 1 oder 2, **dadurch gekennzeichnet, dass** es nach dem Schritt e) einen Schritt des Spülens der das stagnierende Wasser enthaltenden Leitung (1) umfasst, welcher darin besteht, dass man Sanitärwasser in der Leitung zirkulieren lässt, um die veränderten Verunreinigungen durch Ausspülen zu beseitigen.

4. System zur Durchführung des Verfahrens gemäß einem der Ansprüche 1 bis 3, **dadurch gekennzeichnet, dass** es eine Sanitärwasserleitung (1) aufweist, die über ihre gesamte Länge wenigstens eine kontinuierliche leitfähige Schicht (20; 56) aus einem den elektrischen Strom ohnmisch leitenden Material, ein Verbindungselement (5; 59, 71) aus einem den elektrischen Strom leitenden Material, welches zwei Abschnitte (A, B) der leitfähigen Schicht zur Bildung einer geschlossenen Leiterschleife (ABA) verbindet, und eine Vorrichtung zur Erzeugung eines variablen magnetischen Flusses durch die geschlossene Leiterschleife aufweist.

5. System gemäß Anspruch 4, **dadurch gekennzeichnet, dass** die leitfähige Leitung wenigstens ein Rohr (10; 50) mit metallischer Seele (20; 56), die zwischen einer inneren und einer äußeren Schicht eines elektrischen Isolators (21, 22; 55, 59) eingesetzt ist, aufweist, wobei die beiden durch das Verbindungselement verbundenen Abschnitte solchen Abschnitten des Rohrs entsprechen, die von einer Schicht des Isolators befreit wurden, um die metallische Seele freizulegen und einen elektrischen Kontakt zwischen der metallischen Seele und dem Verbindungselement zu ermöglichen.

6. System gemäß Anspruch 5, **dadurch gekennzeichnet, dass** die leitfähige Leitung mehrere Rohre (10; 50) mit metallischer Seele umfasst, die durch Kupplungsstücke miteinander verbunden sind, wobei die elektrische Kontinuität der Leitungsanordnung auf Höhe der Kupplungsstücke mittels elektrischer Zwischenverbindungen gewährleistet wird, welche jeweils die an ein Kupplungsstück angrenzenden freigelegten Enden der auf beiden Seiten des Kupplungsstücks befindlichen Rohre miteinander verbinden.

7. System gemäß Anspruch 5, **dadurch gekennzeichnet, dass** die leitfähige Leitung mehrere Rohre (10; 50) mit metallischer Seele umfasst, die durch leitfähige Kupplungsstücke (10, 28; 52, 113) miteinander verbunden sind, welche eine Schicht aus einem den elektrischen Strom leitenden Material aufweisen.

8. System gemäß Anspruch 7, **dadurch gekennzeichnet, dass** wenigstens ein leitfähiges Kupplungsstück (70; 113) wenigstens eine Schulter (62) aufweist, welche einen elektrischen Kontakt zwischen der leitfähigen Schicht des leitfähigen Kupplungsstücks einerseits und eines Teils der metallischen Seele (56) andererseits ermöglicht, die sich an dem an das Kupplungsstück angrenzenden Ende des Rohrs befindet und von wenigstens einer ihrer Isolierschichten (55) befreit ist.

9. System gemäß Anspruch 7, **dadurch gekennzeichnet, dass** wenigstens ein leitfähiges Kupplungsstück (12, 13) ein externes leitfähiges ringförmiges Bauteil (35) aufweist, welches mit radial nach innen ragenden Spitzen (36) versehen ist, welche die externe Isolierschicht (21) des Rohrs (10) durchqueren und in Kontakt mit der metallischen Seele (20) des Rohrs gelangen und so die elektrische Verbindung zwischen der metallischen Seele des Rohrs und der leitfähigen Schicht des leitfähigen Kupplungsstücks gewährleisten.

10. System gemäß einem der Ansprüche 5 bis 9, **dadurch gekennzeichnet, dass** die Leitung mehrere Rohre (50) umfasst, die eine das Verbindungselement bildende zweite metallische Seele (58) aufweisen, die zwischen der Außenschicht (59) und der ersten metallischen Seele (56) angeordnet und von dieser durch eine zusätzliche für elektrischen Strom isolierende Schicht (57) getrennt ist.

11. System gemäß einem der Ansprüche 4 bis 10, **dadurch gekennzeichnet, dass** die Vorrichtung zur Erzeugung eines variablen magnetischen Flusses durch die Schleife (ABA) ein Transformator (6) mit Magnetkern (8) ist, dessen Primärwicklung (7) durch einen variablen Strom gespeist wird und dessen Sekundärwicklung von der geschlossenen Leiterschleife gebildet wird.

12. System gemäß den kombinierten Ansprüchen 7 und 11, **dadurch gekennzeichnet, dass** wenigstens eines der leitfähigen Kupplungsstücke (52) als Transformatorkupplung einerseits eine innere Wand (70), an der sich die stromleitende Schicht befindet, und andererseits eine äußere Wand (71) aufweist, die mit der inneren Wand einen ringförmigen Raum (85) zwischen den beiden Wänden definiert, in welcher der Magnetkem (8), sowie die Primärwicklung (7) des Transformators (6) angeordnet ist.

13. System gemäß den kombinierten Ansprüchen 10 und 12, **dadurch gekennzeichnet, dass** die Transformatorkupplung (52) in ihrer äußeren Wand (71) eine zweite den elektrischen Strom leitende Schicht in elektrischer Verbindung mit der zweiten metallischen Seele (58) des Rohrs (50) aufweist, wobei die zweiten leitfähigen Schichten der Transformatorkupplung elektrisch voneinander isoliert sind.
